Europäisches Patentamt

European Patent Office

Office européen des brevets

(11) Numéro de publication: **0 328 463**
**A1**

## (12) DEMANDE DE BREVET EUROPEEN

(21) Numéro de dépôt: 89420019.5

(22) Date de dépôt: 26.01.89

(51) Int. Cl.⁴: **A 61 F 2/38**

(30) Priorité: 29.01.88 FR 8801402

(43) Date de publication de la demande:
16.08.89 Bulletin 89/33

(84) Etats contractants désignés:
AT BE CH DE ES GB GR IT LI LU NL SE

(71) Demandeur: **Broc, Christian**
**Les Beaumettes**
**F-84220 Gordes (FR)**

(72) Inventeur: **Broc, Christian**
**Les Beaumettes**
**F-84220 Gordes (FR)**

(74) Mandataire: **Dupuis, François**
**Cabinet Charras 3 Place de l'Hôtel-de-Ville BP 203**
**F-42005 St. Etienne Cédex 1 (FR)**

(54) Implant fémoral pour prothèse bi-compartimentaire d'articulation du genou.

(57) L'invention se rattache au secteur technique des sciences médicales. L'implant (1) est remarquable en ce que les bords latéraux (1b) et (1c) de l'implant fémoral (1) présentent un profil courbe apte à absorber l'effet rotationnel du tibia par rapport aux condyles notamment au condyle interne que est le centre de rotation, ledit profil courbe étant établi à partir du bord postérieur condylien qui est parallèle au plan de coupe tibiale.

FIG.1

EP 0 328 463 A1

Description

## Implant fémoral pour prothèse bi-compartimentaire d'articulation du genou.

L'invention se rattache au secteur technique des sciences médicales.

L'analyse actuelle du genou s'effectue sur deux plans en fonction des charges mécaniques appliquées au genou et selon l'axe global du membre sachant qu'il y a une charge prépondérante interne ou externe selon la déviation axiale en varus ou valgus. Une telle analyse est incomplète par rapport à la cinétique globale et est donc figée au niveau de la charge qui est dite statique, c'est-à-dire lors d'un membre en extension complète et chargé. Il est donc prépondérant de savoir où passe la charge en fonction de l'obliquité des interlignes, avant toute perte de substance osseuse pour déterminer quel est le compartiment de charge statique du genou.

D'une manière fondamentale, il apparaît donc nécessaire de procéder à une analyse de la notion de charge dynamique, c'est-à-dire de tenir compte de la répartition des contraintes au niveau du genou en fonction du déroulement du pas et donc en fonction de l'effet de basculement du poids entre la partie interne et externe du genou. Cela explique son fonctionnement asymétrique et l'apparition d'une arthrose de rotation. Il convient donc d'effectuer une analyse tridimensionnellle.

Le genou est en fait générateur d'une double torsion fémorale et d'une contre-torsion tibiale qui conditionnent son fonctionnement entraînant un effet de balancier au moment de la marche.

Le problème est donc de savoir apprécier la charge dynamique au moment du pas. Il convient donc de dissocier le genou en trois articulations indépendantes qui sont synergiques les unes des autres et qui combinent une charge statique et une charge dynamique.

Dans chaque compartiment, il y a répartition d'une charge dite statique prépondérante et d'une charge dynamique dite prépondérante, ce qui permet de savoir si les contraintes sont absorbées dans chacun des compartiments en respectant le morphotype, c'est-à-dire le degré du balancier.

Ce balancier de la charge est fonction de la rotation du genou ou de la rotation dite pathologique, car il existe aussi une arthrose rotatoire pure.

Cette arthrose rotatoire entraîne un mauvais fonctionnement de la rotation du genou qui se trouve trop mobile, ou bien avec une rotation limitée. Une telle rotation limitée dans le compartiment rotateur va se transférer dans le compartiment de charge dit statique prépondérant et va donc chercher à absorber la rotation de ce compartiment dont ce n'est pas la fonction. Dans ces conditions, il apparaît une arthrose de rotation en plus de l'arthrose de charge telle qu'elle apparaît dans un tibia varum. Il en résulte donc qu'un implant doit permettre d'absorber cette contrainte de rotation dans le plateau interne, sinon ce dernier sera contraint.

Dans le Brevet FR 87.06043 dont le demandeur est aussi titulaire, on décrit les implants tibiaux d'une prothèse bi-compartimentaire qui sont conformés pour résoudre une partie du problème posé, notamment en tenant compte de composants rotatoires résultant de la flexion du genou, au niveau du compartiment externe qui est celui de la mobilité.

La présente invention s'est fixée pour but, toujours en ayant pour objectif de résoudre le problème posé d'absorber les contraintes de rotation, de réaliser une nouvelle forme d'implant fémoral. A ce jour, les implants fémoraux proposés, du type de ceux dont le profil en section correspond au rayon de courbure moyen du condyle, présentent certains inconvénients et ne permettent pas de résoudre le problème posé d'une manière satisfaisante.

En effet, les bords des implants sont rectilignes pour être perpendiculaires aux charges statiques. Pour tenir compte de la rotation au moment de la flexion, il est nécessaire de leur donner une certaine obliquité. Cependant, il existe des phénomènes rotatoires qui sont très importants, de sorte qu'il est nécessaire de "coucher" l'implant. Dans ces conditions, les charges statiques ne sont plus absorbées, ce qui est contraire à la fonction recherchée initialement avec ce type d'implant.

Le problème posé est résolu en ce que les bords latéraux de l'implant fémoral présentent un profil courbe apte à absorber l'effet rotationnel du tibia par rapport aux condyles notamment au condyle interne qui est le centre de rotation, ledit profil courbe étant établi à partir du bord postérieur condylien qui est parallèle au plan de coupe tibiale.

D'une manière avantageuse, l'implant fémoral selon l'invention est particulièrement bien adapté en combinaison avec les éléments tibiaux de la prothèse bi-compartimentaire définie et revendiquée dans le Brevet FR 87.06403.

D'autres caractéristiques permettent d'apporter une solution avantageuse au problème posé, à savoir :

L'implant présente des moyens d'ancrage répartis selon une ligne droite en étant orientés très sensiblement selon la diagonale XX' d'un implant fictif dont les bords latéraux sont rectilignes.

L'implant présente du côté de sa face interne et suivant son axe médian, une ailette verticale de positionnement orientée selon le rayon de courbure des côtés latéraux.

L'implant est remarquable en ce que les moyens d'ancrage sont au nombre de trois en étant disposés à chacune des extrémités de la diagonale et dans sa partie médiane.

L'implant est également remarquable en ce que l'ailette est établie à mi-hauteur du rebord condylien et rejoint la partie antérieure d'une manière inclinée et dégressive.

L'invention est exposée ci-après plus en détail à l'aide des dessins annexés dans lesquels :

La figure 1 est une vue de face de l'implant.

La figure 2 est une vue en coupe considérée selon la ligne 2-2 de la figure 1.

La figure 3 montre l'implantation de l'élément prothétique fémoral sur le condyle externe, le genou étant représenté en position de flexion.

La figure 4 montre l'effet rotationnel du tibia par rapport aux condyles fémoraux, et la fonction remplie par l'implant dans ces conditions.

Afin de rendre plus concret l'objet de l'invention, on le décrit maintenant d'une manière non limitative en se référant aux exemples de réalisation des figures des dessins.

D'une manière connue, l'implant fémoral désigné dans son ensemble par (1), a, dans un plan latéral, un rayon de courbure moyen (1a) correspondant à celui du condyle externe où il doit être posé.

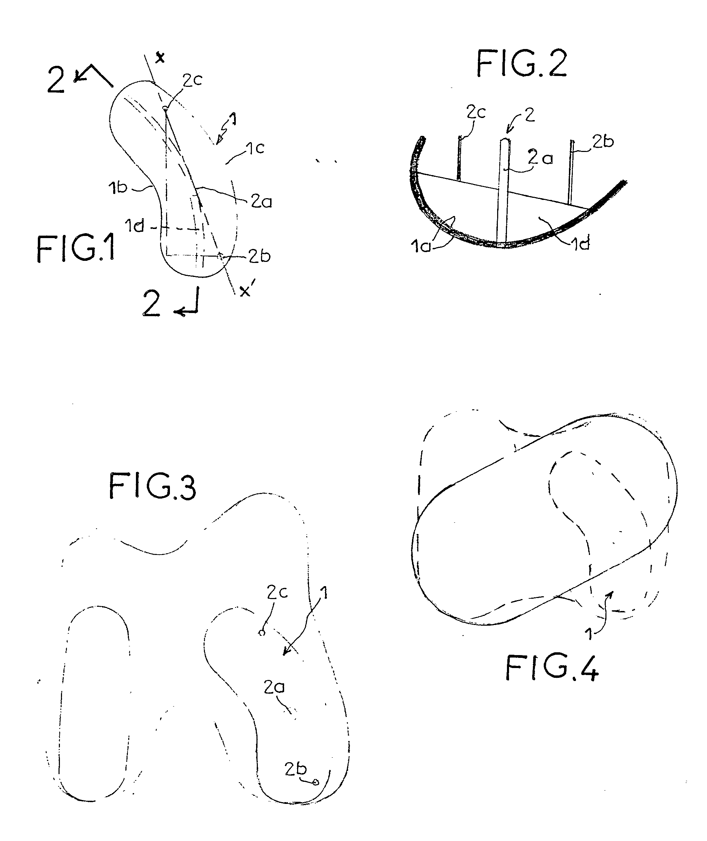

De même, le profil transversal est très légèrement convexe pour constituer en combinaison avec le rayon de courbure (1a) une surface d'appui et de glissement apte à coopérer avec un implant tibial (A) disposé dans le compartiment correspondant du tibia (figure 3).

Selon l'invention, les bords latéraux (1b) et (1c) de l'implant (1), présentent un profil courbe apte à absorber l'effet rotationnel du tibia par rapport aux condyles fémoraux, notamment du condyle interne qui est le centre de rotation (figure 4). Le profil courbe de chacun des côtés (1b) et (1c) est établi à partir du bord postérieur conduylien qui est parallèle au plan des coupes tibiales, afin de pouvoir toujours absorber les charges statiques.

Ainsi réalisé, l'implant présente des moyens d'ancrage (2) qui sont répartis selon un même alignement et orientés très sensiblement selon la diagonale XX' d'un implant fictif à bords latéraux parallèles et rectilignes en direction de l'échancrure intercondylienne (figure 2). D'une manière préférée, ces moyens d'ancrage (2) sont au nombre de trois en étant disposés à chacune des extrémités de la diagonale XX' et dans sa partie médiane. Les moyens d'ancrage (2) sont constitués par des plots, de conception connue et appropriée.

Suivant une autre caractéristique, la face interne de l'implant (1) présente selon son axe médian, une ailette verticale de positionnement (1d) orientée selon le même rayon de courbure que celui des côtés latéraux. Le plot central d'ancrage (2a) passe par cette ailette, tandis que les plots d'extrémité (2b) et (2c) sont situés d'un même côté à l'extérieur de ladite ailette.

Cette ailette (1d) est en outre établie très sensiblement à mi-hauteur du rebord postérieur condylien pour rejoindre la partie antérieure d'une manière inclinée et dégressive (figure 1).

La mise en place de l'implant (1) sur le condyle externe de rotation, s'effectue par tout moyen connu et approprié.

A noter que l'autre condyle, c'est-à-dire le condyle interne reçoit un implant fémoral de conception classique, à bords rectilignes et parallèles.

## Revendications

-1- Implant fémoral dont le profil en section correspond au rayon de courbure moyen du condyle externe, caractérisé en ce que les bords latéraux de l'implant fémoral (1b) et (1c) présentent un profil courbe apte à absorber l'effet rotationnel du tibia par rapport aux condyles notamment au condyle interne que est le centre de rotation, ledit profil courbe étant établi à partir du bord postérieur condylien qui est parallèle au plan de coupe tibiale.

-2-Implant selon la revendication 1, caractérisé en ce qu'il présente des moyens d'ancrage (2) répartis selon une ligne droite en étant orientés très sensiblement selon la diagonale XX' d'un implant fictif dont les bords latéraux sont rectilignes.

-3- Implant selon la revendication 1, caractérisé en ce qu'il présente du côté de sa face interne et suivant son axe médian, une ailette verticale de positionnement (1d) orientée selon le rayon de courbure des côtés latéraux (1b) et (1c).

-4- Implant section la revendication 2, caractérisé en ce que les moyens d'ancrage (2) sont au nombre de trois en étant disposés à chacune des extrémités de la diagonale et de sa partie médiane.

-5- Implant selon la revendication 3, caractérisé en ce que l'ailette (1d) est établie à mi-hauteur du rebord condylien et rejoint la partie antérieure d'une manière inclinée et dégressive.

FIG.1

FIG.2

FIG.3

FIG.4

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y | FR-A-2 589 720 (AUBANIAC) * Page 3, ligne 34 - page 4, ligne 1; page 4, lignes 5-14,18-20; figures 13-16 * | 1,2 | A 61 F 2/38 |
| Y | FR-A-2 136 541 (HELFET) * Page 5, ligne 12 - page 6, ligne 2; page 6, lignes 9-16,23-40; figures 3-6,9,10 * | 1,2 | |
| A | FR-A-2 417 293 (HELFET) * Page 4, ligne 12 - page 5, ligne 18; figures 4-6 * | 1,2 | |
| A | FR-A-2 478 462 (JUDET) * Page 8, lignes 13-24; figures 16,17 * | 1,2 | |
| A | MED.-ORTHOP.-TECHN., vol. 105, no. 2, mars-avril 1985, pages 49-53, Stuttgart, DE; D. TÖNNIS: "Die Schlittenprothese (Modell nach Tönnis) Implantationstechnik, Indikation, Ergebnisse" * Figure 1 * | 2,3,5 | DOMAINES TECHNIQUES RECHERCHES (Int. Cl.4) |
| A | US-A-3 852 830 (MARMOR) * Colonne 3, ligne 63 - colonne 4, ligne 26; figures 1-3 * | 2,3,5 | A 61 F |
| A | WO-A-8 603 117 (HANSLIK) | | |
| A,D P | EP-A-0 288 402 (BROC) | | |

Le présent rapport a été établi pour toutes les revendications

| Lieu de la recherche | Date d'achèvement de la recherche | Examinateur |
|---|---|---|
| LA HAYE | 28-04-1989 | KLEIN C. |